(19) Europäisches Patentamt
European Patent Office
Office européen des brevets

(11) EP 4 722 706 A1

(12) **EUROPEAN PATENT APPLICATION**
published in accordance with Art. 153(4) EPC

(43) Date of publication:
**08.04.2026 Bulletin 2026/15**

(21) Application number: **24815081.5**

(22) Date of filing: **30.04.2024**

(51) International Patent Classification (IPC):
***G01N 27/72*** *(2006.01)*

(52) Cooperative Patent Classification (CPC):
**G01N 27/72**

(86) International application number:
**PCT/JP2024/016706**

(87) International publication number:
**WO 2024/247597 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.06.2023 JP 2023090957**

(71) Applicant: **MITSUBISHI ELECTRIC CORPORATION**
**Chiyoda-ku**
**Tokyo 100-8310 (JP)**

(72) Inventors:
- **NOMURA, Kota**
  **Tokyo 100-8310 (JP)**
- **WASHINO, Masaomi**
  **Tokyo 100-8310 (JP)**

(74) Representative: **Meissner Bolte Partnerschaft mbB**
**Patentanwälte Rechtsanwälte**
**Postfach 86 06 24**
**81633 München (DE)**

(54) **DETECTION METHOD, DETECTION SYSTEM, PROGRAM, AND RECORDING MEDIUM**

(57) An information processing device (9) of a detection system (100) acquires a first phase of a first signal of a specific frequency extracted from a magnetic signal when a first magnetic particle is placed in a target region, and acquires a second phase of a second signal of the specific frequency extracted from the magnetic signal when a second magnetic particle is placed in the target region. A relaxation time of a magnetic moment of the second magnetic particle is different from a relaxation time of a magnetic moment of the first magnetic particle at the specific frequency. The information processing device (9) further acquires a third signal of the specific frequency extracted from the magnetic signal when an inspection object (6) including the first magnetic particle and the second magnetic particle is placed in the target region. The information processing device (9) discriminates a target component of a target particle, of the first magnetic particle and the second magnetic particle, from the third signal by using the first phase and the second phase.

FIG.1

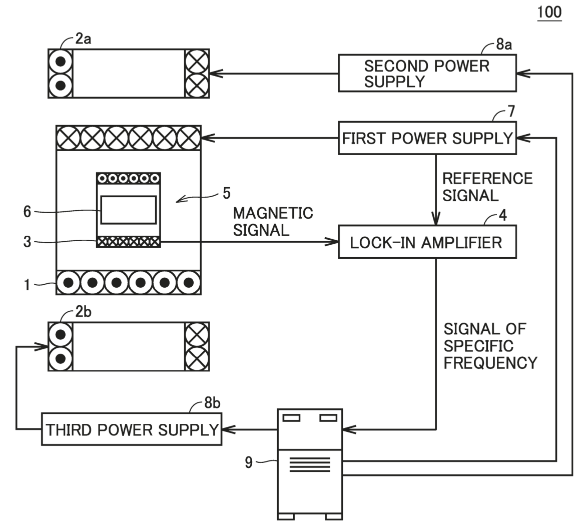


EP 4 722 706 A1

## Description

TECHNICAL FIELD

The present disclosure relates to a detection method, a detection system, a program, and a recording medium for detecting a magnetic particle.

BACKGROUND ART

A detection device that includes an alternating current (AC) magnetic field applicator and a direct current (DC) magnetic field generator and detects a magnetic particle has been conventionally known. Only magnetic particles in a zero magnetic field region generated by the DC magnetic field generator fluctuate in magnetization due to an AC excitation magnetic field applied by the AC magnetic field applicator. Therefore, by scanning the zero magnetic field region in an inspection object, distribution information about the magnetic particles in the inspection object is obtained.

A magnetic signal indicating magnetization fluctuation of magnetic particles includes signals having various frequencies. A signal caused by the magnetic particles and a signal excited by an excitation magnetic field coexist in a signal having the same frequency as that of the excitation magnetic field. Therefore, the signal having the same frequency as that of the excitation magnetic field has a low SN ratio. Thus, a harmonic signal having a high SN ratio is usually used.

As a method for extracting the harmonic signal from the magnetic signal, a method using a frequency filter, a method using fast Fourier transform, or a method for synchronous detection using a lock-in amplifier has been known. Japanese Patent Laying-Open No. 2003-199767 (PTL 1) discloses a method using a frequency filter. "Gleich, B, Weizenecker, J, "Tomographic imaging using the nonlinear response of magnetic particles", nature, Vol. 435, 2005, pp. 1214-1217" (NPL 1) discloses a method using fast Fourier transform. "Kenya Murase et al, "Development of a system for magnetic particle imaging using neodymium magnets and gradiometer", Japanese Journal of Applied Physics, 53, 067001(2014)" (NPL 2) discloses a method for synchronous detection using a lock-in amplifier.

CITATION LIST

PATENT LITERATURE

PTL 1: Japanese Patent Laying-Open No. 2003-199767

NON PATENT LITERATURE

NPL 1: Gleich, B, Weizenecker, J, "Tomographic imaging using the nonlinear response of magnetic particles", nature, Vol. 435, 2005, pp. 1214-1217

NPL 2: Kenya Murase et al, "Development of a system for magnetic particle imaging using neodymium magnets and gradiometer", Japanese Journal of Applied Physics, 53, 067001(2014)

SUMMARY OF INVENTION

TECHNICAL PROBLEM

The techniques disclosed in PTL 1, NPL 1 and NPL 2 are premised on obtainment of distribution information of one type of magnetic particle. Therefore, the techniques disclosed in PTL 1, NPL 1 and NPL 2 cannot be applied to discrimination of a component of one of two magnetic particles.

The present disclosure has been made in view of the above-described problem and an object thereof is to provide a detection method, a detection system, a program, and a recording medium that enable discrimination of a component of one of two magnetic particles.

SOLUTION TO PROBLEM

A detection method according to an aspect of the present disclosure detects a magnetic particle by using an AC excitation magnetic field. The detection method includes: acquiring a first phase of a first signal of a specific frequency generated in response to a change in magnetic moment of a first magnetic particle when the excitation magnetic field is applied; and acquiring a second phase of a second signal of the specific frequency generated in response to a change in magnetic moment of a second magnetic particle when the excitation magnetic field is applied. A relaxation time of the magnetic moment of the second magnetic particle is different from a relaxation time of the magnetic moment of the first magnetic particle at the specific frequency. The detection method further includes: acquiring a third signal of the specific frequency generated in response to a change in magnetic moment of a target region including the first magnetic particle and the second magnetic particle when the excitation magnetic field is applied; and discriminating a target component of a target particle, of the first magnetic particle and the second magnetic particle, from the third signal by using the first phase and the second phase.

A detection system according to an aspect of the present disclosure includes: an excitation magnetic field applicator that applies an AC excitation magnetic field to a target region; a magnetic sensor that detects a magnetic signal indicating a change in magnetic moment of the target region; and a processor. The processor acquires a first phase of a first signal of a specific frequency extracted from the magnetic signal when a first magnetic particle is placed in the target region. The processor acquires a second phase of a second signal of the specific frequency extracted from the magnetic signal when a second magnetic particle is placed in the target region. A relaxation time of a magnetic moment of the second magnetic particle is different from a relaxation time of a magnetic moment of the first magnetic particle at the specific frequency. The processor further acquires a third signal of the specific frequency extracted from the magnetic signal when an inspection object including the first magnetic particle and the second magnetic particle is placed in the target region. The processor discriminates a target component of a target particle, of the first magnetic particle and the second magnetic particle, from the third signal by using the first phase and the second phase.

A program according to an aspect of the present disclosure causes a computer to perform the above-described detection method. A computer-readable recording medium according to an aspect of the present disclosure has the above-described program recorded thereon.

ADVANTAGEOUS EFFECTS OF INVENTION

The detection method, the detection system, the program, and the recording medium according to the present disclosure enable discrimination of a component of one of two magnetic particles by using a phase difference based on a difference between the relaxation times of the magnetic moments of the two magnetic particles.

BRIEF DESCRIPTION OF DRAWINGS

Fig. 1 is a view showing an example of an overall configuration of a detection system according to a first embodiment.
Fig. 2 is a perspective view showing a part of the detection system.
Fig. 3 is a view showing magnetic particles in an inspection object.
Fig. 4 is a view showing the two types of magnetic particles according to the first embodiment.
Fig. 5 is a view showing a configuration of a lock-in amplifier.
Fig. 6 is a view showing an example of a hardware configuration of an information processing device.
Fig. 7 is a flowchart showing an example of a flow of a process in a preliminary preparation phase.
Fig. 8 is a view illustrating processing in step S2.
Fig. 9 is a view illustrating processing in step S5.
Fig. 10 is a flowchart showing an example of a flow of a process in a measurement phase.
Fig. 11 is a view illustrating the process in the measurement phase.
Fig. 12 is a flowchart showing an example of a flow of spatial distribution imaging.
Fig. 13 is a view showing examples of user interface screens provided by the detection system shown in Fig. 1.
Fig. 14 is a view showing two types of magnetic particles detected by a detection system according to a fifth embodiment.

DESCRIPTION OF EMBODIMENTS

Embodiments of the present disclosure will be described in detail hereinafter with reference to the drawings. In the drawings, the same or corresponding portions are denoted by the same reference characters and description thereof will not be repeated. The embodiments and modifications described below may be selectively combined as appropriate.

First Embodiment.

(Overall Configuration of Detection System)

Fig. 1 is a view showing an example of an overall configuration of a detection system according to a first embodiment. A detection system 100 shown in Fig. 1 includes an excitation magnetic field applicator 1, a zero magnetic field generator 2, a magnetic sensor 3, a lock-in amplifier 4, a first power supply 7, a second power supply 8a, a third power supply 8b, and an information processing device 9.

Excitation magnetic field applicator 1 applies an AC excitation magnetic field to an area where an inspection object 6 is placed (hereinafter referred to as "measurement area 5"). Although inspection object 6 may include, for example, a human body, an animal and the like, inspection object 6 is not limited thereto. Specifically, excitation magnetic field applicator 1 is configured of a coil connected to first power supply 7. When a current flows from first power supply 7 to excitation magnetic field applicator 1, the excitation magnetic field is applied to measurement area 5.

Zero magnetic field generator 2 forms a zero magnetic field region in measurement area 5. Specifically, zero magnetic field generator 2 includes a pair of electromagnets 2a and 2b arranged to face each other such that directions of magnetization are opposite to each other. Electromagnets 2a and 2b are connected to second power supply 8a and third power supply 8b, respectively. When currents flow from second power supply 8a and third power supply 8b to electromagnets 2a and 2b, the zero magnetic field region is generated.

In the present embodiment, the case where zero magnetic field generator 2 includes electromagnets 2a and 2b will be described. However, zero magnetic field generator 2 may use two permanent magnets or a combination of a permanent magnet and an electromagnet arranged to face each other instead of electromagnets 2a and 2b. When the zero magnetic field region is formed by the two permanent magnets, second power supply 8a and third power supply 8b are omitted.

Magnetic sensor 3 detects a magnetic signal indicating a change in magnetic moment of a magnetic particle existing in the zero magnetic field region when the excitation magnetic field is applied.

Lock-in amplifier 4 extracts a signal of a specific frequency from the magnetic signal. The specific frequency is a frequency of a reference signal generated by first power supply 7. The reference signal is a high-order harmonic ($m \times f0$) (m is an integer equal to or more than 2) signal of a fundamental wave f0 having the same frequency as that of the excitation magnetic field. That is, the specific frequency is a frequency that is m times as high as the frequency of the excitation magnetic field. The signal extracted by lock-in amplifier 4 is output to information processing device 9.

Information processing device 9 is connected to each unit of detection system 100 through a bus. Information processing device 9 executes various types of information processing for controlling the operation of detection system 100. Information processing device 9 detects the magnetic particle existing in the zero magnetic field region, based on the signal extracted by lock-in amplifier 4.

(Zero Magnetic Field Region)

Fig. 2 is a perspective view showing a part of the detection system. In the example shown in Fig. 2, a pair of electromagnets 2a and 2b included in zero magnetic field generator 2 generates a field free line (FFL) 30. However, in the present embodiment, a shape of a zero magnetic field region 30 is not limited to the linear shape. For example, zero magnetic field region 30 may be a field free point (FFP).

Zero magnetic field region 30 may be scanned in inspection object 6. Specifically, the position and direction of zero magnetic field region 30 are scanned. The position of zero magnetic field region 30 is indicated by, for example, a position on an axis 70 of a coordinate system set in detection system 100 (hereinafter referred to as "scanning position"). The direction of zero magnetic field region 30 is indicated by, for example, an angle $\phi$ formed by an axis 72 of the coordinate system set in detection system 100 and zero magnetic field region 30. A method for scanning zero magnetic field region 30 in inspection object 6 may include a method for mechanically moving zero magnetic field generator 2, a method for changing a current balance of electromagnets 2a and 2b, or a method for mechanically moving inspection object 6.

(Magnetic Particles)

Fig. 3 is a view showing magnetic particles in the inspection object. As shown in Fig. 3, two types of magnetic particles 51 and 52 are introduced into inspection object 6. A magnetic particle 51 is an example of "first magnetic particle" in the present disclosure. A magnetic particle 52 is an example of "second magnetic particle" in the present disclosure. Magnetic particle 51 and magnetic particle 52 are also referred to as "first type of magnetic particle" and "second type of magnetic particle", respectively.

When the AC excitation magnetic field is applied to inspection object 6 including magnetic particles 51 and 52, magnetic moments of only magnetic particles 51 and 52 existing in zero magnetic field region 30 fluctuate due to the excitation magnetic field. Therefore, zero magnetic field region 30 corresponds to "target region" where changes in magnetic moments of magnetic particles 51 and 52 are detected by magnetic sensor 3.

A relaxation time of the magnetic moment of magnetic particle 52 when the excitation magnetic field is applied is different from a relaxation time of the magnetic moment of magnetic particle 51 when the excitation magnetic field is applied.

Fig. 4 is a view showing the two types of magnetic particles according to the first embodiment. As shown in Fig. 4, in the first embodiment, magnetic particles 51 and 52 have different particle sizes. When the Brownian motion is not dominant in magnetic particles 51 and 52, Neel relaxation times of magnetic particles 51 and 52 are different from each other because magnetic particles 51 and 52 have different particle sizes.

A probe 51a having the property of binding to a first protein is attached to magnetic particle 51. A probe 52a having the property of binding to a second protein is attached to magnetic particle 52. When a certain period of time elapses since magnetic particles 51 and 52 were introduced into inspection object 6, magnetic particles 51 and 52 bind to the first and second proteins existing in a tissue 60 in inspection object 6 through probes 51a and 52a, respectively. Therefore, when zero magnetic field region 30 is generated in inspection object 6 after floating magnetic particles 51 and 52 are discharged from inspection object 6, magnetic signals indicating the changes in magnetic moments of magnetic particles 51 and 52 binding to the first and second proteins, respectively, are obtained in zero magnetic field region 30. By discriminating a component of each of magnetic particles 51 and 52 from the magnetic signals, an amount of each of magnetic particles 51 and 52 existing in zero magnetic field region 30 can be estimated.

(Configuration of Lock-In Amplifier)

Fig. 5 is a view showing a configuration of the lock-in amplifier. As shown in Fig. 5, lock-in amplifier 4 includes mixers 41 and 42, low-pass filters 43 and 44, and a calculator 45. Lock-in amplifier 4 receives a magnetic signal $V_s(t)$ from magnetic sensor 3 and receives a reference signal $V_r(t)$ of the specific frequency from first power supply 7.

Mixer 41 multiplies magnetic signal $V_s(t)$ by reference signal $V_r(t)$, thereby outputting an X component. Low-pass filter 43 filters the X component output from mixer 41.

Mixer 42 multiplies magnetic signal $V_s(t)$ by a signal obtained by shifting a phase of reference signal $V_r(t)$ by 90°, thereby outputting a Y component. Low-pass filter 44 filters the Y component output from mixer 42.

Mixers 41 and 42 constitute a so-called dual-phase demodulation circuit. The dual-phase demodulation circuit outputs, as the X component, a real part Re(Z) of a complex signal Z(t), which is a product of magnetic signal $V_s(t)$ indicated by a complex number and reference signal $V_r(t)$ indicated by a complex number, and outputs an imaginary part Im(Z) of complex signal Z(t) as the Y component.

As shown in the following equation (1), magnetic signal $V_s(t)$ is rewritten into a sum of two vectors on a complex plane that rotate at the same speed $\omega_s$ and each have a length $R/(2^{1/2})$. R represents an intensity (effective value) of magnetic signal $V_s(t)$. One of the two vectors rotates clockwise and the other rotates counterclockwise.

[Math. 1]

$$\begin{aligned} \mathrm{V_s}(t) &= \sqrt{2}R\cos(\omega_s t+\theta) \\ &= \frac{R}{\sqrt{2}}e^{+i(\omega_s t+\theta)} + \frac{R}{\sqrt{2}}e^{-i(\omega_s t+\theta)} \qquad (1) \end{aligned}$$

Reference signal $V_r(t)$ has a specific frequency $\omega_r$ that is m times as high as the frequency of the excitation magnetic field. Phasor notation of reference signal $V_r(t)$ is expressed by the following equation (2). Phasor notation of complex signal Z(t) is expressed by the following equation (3).

[Math. 2]

$$\mathrm{V_r}(t) = \sqrt{2}e^{-i\omega_r t} \qquad (2)$$

$$\begin{aligned} Z(t) &= V_s(t)\cdot V_r(t) \\ &= R\left[e^{i\{\omega_s-\omega_r\}t+\theta} + e^{-i\{\omega_s+\omega_r\}t+\theta}\right] \qquad (3) \end{aligned}$$

In the equation (3), the second term is a high-speed rotation term that rotates at a frequency $(\omega_s+\omega_r)$. Therefore, the second term becomes zero through low-pass filters 43 and 44. Furthermore, when $\omega_s$ does not match $\omega_r$, the first term also becomes zero through low-pass filters 43 and 44. Therefore, after passing through low-pass filters 43 and 44, complex signal Z(t) is expressed by the following equation (4).

$$Z(t) = R \bullet e^{i\theta} = R\cos\theta + iR\sin\theta \qquad (4)$$

Therefore, the X component output from low-pass filter 43 is indicated by $R\cos\theta$, which is a real part of Z(t). The Y component output from low-pass filter 44 is indicated by $R\sin\theta$, which is an imaginary part of Z(t).

By using the X component and the Y component output from low-pass filters 43 and 44, calculator 45 calculates R and $\theta$ in accordance with the following equations (5) and (6). R represents an intensity (effective value) of a signal having specific frequency $\omega_r$, of the magnetic signal. $\theta$ represents a phase of the signal having specific frequency $\omega_r$, of the magnetic signal, with respect to the reference signal.

[Math. 3]

$$R = \sqrt{X^2 + Y^2} \qquad (5)$$

$$\theta = \tan^{-1}\left(\frac{Y}{X}\right) \qquad (6)$$

In this way, lock-in amplifier 4 extracts, from the magnetic signal received from magnetic sensor 3, the signal of the specific frequency generated in response to a change in magnetic moment of the magnetic particle in zero magnetic field region 4, and specifies intensity R and phase $\theta$ of the extracted signal. Lock-in amplifier 4 outputs, to information processing device 9, intensity R and phase $\theta$ that define the signal of the specific frequency extracted from the magnetic signal.

(Hardware Configuration of Information Processing Device)

Fig. 6 is a view showing an example of a hardware configuration of the information processing device. As shown in Fig. 6, information processing device 9 includes a processor 12, a random access memory (RAM) 13, a reading unit 14, an internal storage unit 15, a display unit 16, an operation unit 17, and a communication interface 18.

For example, processor 12 is a central processing unit (CPU), and executes arithmetic processing. RAM 13 stores temporary information generated in accordance with the arithmetic processing of processor 12. Processor 12 reads a program (including detection program 10) stored in internal storage unit 15, loads the program in RAM 13, and executes the program.

Reading unit 14 reads information recorded on an optical recording medium 11 such as a compact disk read only memory (CD-ROM).

For example, internal storage unit 15 is a hard disk drive, and stores various programs such as detection program 10 and various types of data. Furthermore, internal storage unit 15 stores phase information 20. Phase information 20 is generated by execution of detection program 10 and used for execution of detection program 10.

For example, display unit 16 is a liquid crystal display, and displays a screen generated according to the arithmetic processing of processor 12. For example, operation unit 17 includes a keyboard, a mouse, and the like, and receives an operation input by an operator.

Communication interface 18 communicates with an external device (for example, a server device 19) through a network. For example, server device 19 accumulates results of processing by information processing device 9.

Detection program 10 includes a command group of processing related to detection of the magnetic particle. For example, detection program 10 is recorded in optical recording medium 11, read by reading unit 14, and stored in internal storage unit 15. Alternatively, detection program 10 may be downloaded from server device 19 by communication interface 18 and stored in internal storage unit 15.

(Flow of Detection Method for Detecting Magnetic Particles)

A detection method for detecting magnetic particles 51 and 52 in detection system 100 includes a preliminary preparation phase and an actual measurement phase.

<Preliminary Preparation Phase>

Fig. 7 is a flowchart showing an example of a flow of a process in the preliminary preparation phase. First, in steps S1 to S3, a first phase of a first signal of a specific frequency generated in response to a change in magnetic moment of magnetic particle 51, which is an example of the first magnetic particle, when an excitation magnetic field is applied is acquired.

Specifically, in step S1, processor 12 of information processing device 9 causes display unit 16 to display a screen that encourages placing magnetic particle 51, which is an example of the first magnetic particle, in measurement area 5. Upon receipt of an input indicating that placement of magnetic particle 51 in measurement area 5 has been completed, processor 12 instructs first power supply 7, second power supply 8a and third power supply 8b to supply electric power. As a result, zero magnetic field region 30 is formed in measurement area 5 and an AC excitation magnetic field is applied. Furthermore, consequently, magnetic sensor 3 measures a magnetic signal indicating a change in magnetic moment of magnetic particle 51 existing in zero magnetic field region 30.

In step S2, lock-in amplifier 4 extracts, from the magnetic signal measured in step S1, the first signal of the specific frequency generated in response to the change in magnetic moment of magnetic particle 51, and specifies an intensity $R_1$ and a phase $\theta_1$ of the first signal. Phase $\theta_1$ is an example of "first phase" in the present disclosure.

Fig. 8 is a view illustrating the processing in step S2. As shown in Fig. 8, a point 91 having an X component $X_1$ and a Y component $Y_1$ output from low-pass filters 43 and 44 of lock-in amplifier 4 is expressed in polar coordinate, whereby intensity $R_1$ and phase $\theta_1$ of the first signal are specified.

Returning to Fig. 7, in step S3, processor 12 acquires intensity $R_1$ and phase $\theta_1$ of the first signal from the lock-in amplifier.

In next steps S4 to S6, a second phase of a second signal of the specific frequency generated in response to a change in magnetic moment of magnetic particle 52, which is an example of the second magnetic particle, when the excitation magnetic field is applied is acquired.

Specifically, in step S4, processor 12 of information processing device 9 causes display unit 16 to display a screen that encourages placing magnetic particle 52 in measurement area 5. Upon receipt of an input indicating that placement of magnetic particle 52 in measurement area 5 has been completed, processor 12 instructs first power supply 7, second power supply 8a and third power supply 8b to supply electric power. As a result, zero magnetic field region 30 is formed in measurement area 5 and the AC excitation magnetic field is applied. Consequently, magnetic sensor 3 measures a magnetic signal indicating a change in magnetic moment of magnetic particle 52 existing in zero magnetic field region 30.

In step S5, lock-in amplifier 4 extracts, from the magnetic signal measured in step S4, the second signal of the specific frequency generated in response to the change in magnetic moment of magnetic particle 52, and specifies an intensity $R_2$ and a phase $\theta_2$ of the second signal. Phase $\theta_2$ is an example of "second phase" in the present disclosure.

Fig. 9 is a view illustrating the processing in step S5. As shown in Fig. 9, a point 92 having an X component $X_2$ and a Y component $Y_2$ output from low-pass filters 43 and 44 of lock-in amplifier 4 is expressed in polar coordinate, whereby intensity $R_2$ and phase $\theta_2$ of the second signal are specified.

Returning to Fig. 7, in step S6, processor 12 acquires intensity $R_2$ and phase $\theta_2$ of the second signal from the lock-in amplifier.

In step S7, processor 12 calculates a phase difference $\Delta\theta$ (= $\theta_1$-$\theta_2$) between phase $\theta_1$ and phase $\theta_2$.

In step S8, processor 12 determines rotation angles $\theta_{rot1}$ and $\theta_{rot2}$. Rotation angle $\theta_{rot1}$ is a rotation angle centered at the origin for moving point 92 (see Fig. 8) onto the X axis or the Y axis. Rotation angle $\theta_{rot2}$ is a rotation angle centered at the origin for moving point 91 (see Fig. 7) onto the X axis or the Y axis. Rotation angles $\theta_{rot1}$ and $\theta_{rot2}$ are expressed by the following equations (7) and (8), respectively:

$$\theta_{rot1} = -\theta_2 + (\pi/2) \times n \qquad (7)$$

$$\theta_{rot2} = -\theta_1 + (\pi/2) \times n \qquad (8),$$

where n is an integer equal to or more than 0. When n is an even number, points 91 and 92 move onto the X axis. When n is an odd number, points 91 and 92 move onto the Y axis.

In step S9, processor 12 generates phase information 20 indicating phase difference $\Delta\theta$ and rotation angles $\theta_{rot1}$ and $\theta_{rot2}$, and stores phase information 20 in internal storage unit 15.

<Measurement Phase>

A flow of a process in the measurement phase will be described with reference to Figs. 10 and 11. Fig. 10 is a flowchart showing an example of the flow of the process in the measurement phase. Fig. 11 is a view illustrating the process in the measurement phase. In the measurement phase, magnetic particles 51 and 52 are introduced into inspection object 6 in advance.

In step S11, upon receipt of an input indicating that placement of inspection object 6 in measurement area 5 has been completed, processor 12 of information processing device 9 instructs first power supply 7, second power supply 8a and third power supply 8b to supply electric power. As a result, zero magnetic field region 30 is formed in measurement area 5 and the AC excitation magnetic field is applied. Consequently, magnetic sensor 3 measures a magnetic signal indicating a change in magnetic moment of zero magnetic field region 30 in inspection object 6. Specifically, magnetic sensor 3 measures the magnetic signals indicating the changes in magnetic moments of magnetic particles 51 and 52 existing in zero magnetic field region 30.

In step S12, lock-in amplifier 4 extracts, from the magnetic signal measured in step S11, a third signal of specific frequency $\omega_r$ generated in response to the change in magnetic moment of zero magnetic field region 30. The third signal is expressed as $2^{2/1}R_{3cos}(\omega_r t+\theta_3)$ by using specific frequency $\omega_r$, an intensity $R_3$ and a phase $\theta_3$. Processor 12 acquires the third signal from lock-in amplifier 4. Specifically, processor 12 acquires intensity $R_3$ and phase $\theta_3$ that define the third signal.

As shown in Fig. 11, in a polar coordinate, a point 93 having the coordinate $(2^{2/1}R_3, \theta_3)$ indicates the third signal. The third signal is a sum of a first component caused by the change in magnetic moment of magnetic particle 51 and a second component caused by the change in magnetic moment of magnetic particle 52. That is, a vector $V_3$ starting from the origin and ending at point 93 is a sum of a vector $V_1$ corresponding to the first component and a vector $V_2$ corresponding to the second component. Vector $V_1$ has a direction whose angle is phase $\theta_1$ in the polar coordinate. Vector $V_2$ has a direction whose angle is phase $\theta_2$ in the polar coordinate.

Returning to Fig. 10, in step S13, processor 12 selects a target particle from magnetic particles 51 and 52 in accordance with an input to operation unit 17. Hereinafter, a particle that is not selected, of magnetic particles 51 and 52, will be referred to as "the other magnetic particle". In the example shown in Fig. 11, processor 12 selects magnetic particle 51 as the target particle.

Returning to Fig. 10, in steps S14 to S16, processor 12 discriminates a component of the target particle (hereinafter referred to as "target component"), of magnetic particles 51 and 52, from the third signal by using phase $\theta_1$ and phase $\theta_2$. That is, when magnetic particle 51 is selected as the target particle, processor 12 specifies a size of vector $V_1$ (see Fig. 11) corresponding to the first component. When magnetic particle 52 is selected as the target particle, processor 12 specifies a size of vector $V_2$ (see Fig. 11) corresponding to the second component.

Specifically, in step S14, processor 12 reads a rotation angle corresponding to the target particle from phase information 20 stored in internal storage unit 15. When magnetic particle 51 is selected as the target particle, processor 12 reads rotation angle $\theta_{rot1}$. When magnetic particle 52 is selected as the target particle, processor 12 reads rotation angle $\theta_{rot2}$. Processor 12 performs rotation processing on the third signal by the rotation angle.

In the example shown in Fig. 11, magnetic particle 51 is selected as the target particle, and thus, processor 12 reads rotation angle $8_{rot1}$ (= $-\theta_2$). Processor 12 rotates point 93 indicating the third signal around the origin by read rotation angle $\theta_{rot1}$.

As a result of the rotation processing, a vector corresponding to a component of the other magnetic particle is located on the X axis or the Y axis. When n is an even number in the equations (7) and (8) above, the vector corresponding to the component of the other magnetic particle is located on the X axis. When n is an odd number in the equations (7) and (8) above, the vector corresponding to the component of the other magnetic particle is located on the Y axis. In the example shown in Fig. 11, n = 0 and magnetic particle 51 is selected as the target particle, and thus, vector $V_2$ corresponding to magnetic particle 52 is located on the X axis.

In next step S15, processor 12 calculates an intensity Ra of a component having a phase orthogonal to the phase of the other magnetic particle in the third signal. That is, when the target particle is magnetic particle 51, processor 12 calculates an intensity $Ra_1$ of a component having a phase orthogonal to phase $\theta_2$ in the third signal. When the target particle is magnetic particle 52, processor 12 calculates an intensity $Ra_2$ of a component having a phase orthogonal to phase $\theta_1$ in the third signal.

As described above, in the example shown in Fig. 11, vector $V_2$ corresponding to magnetic particle 52 is located on the X axis. Therefore, processor 12 determines, as intensity $Ra_1$, an absolute value of the Y coordinate of point 93 subjected to the rotation processing. When vector $V_2$ corresponding to magnetic particle 52 is located on the Y axis, processor 12 may determine, as intensity $Ra_1$, an absolute value of the X coordinate of point 93 subjected to the rotation processing.

In next step S16, processor 12 calculates a product Rb of intensity Ra and 1/sin ($\Delta\theta$) as the target component of the target particle. That is, when the target particle is magnetic particle 51, processor 12 calculates a product $Rb_1$ of intensity $Ra_1$ and 1/sin ($\Delta\theta$) as the target component. When the target particle is magnetic particle 52, processor 12 calculates a product $Rb_2$ of intensity $Ra_2$ and 1/sin ($\Delta\theta$) as the target component.

In the example shown in Fig. 11, product $Rb_1$ of intensity $Ra_1$ and 1/sin ($\Delta\theta$) is calculated as the target component. Product $Rb_1$ represents the size of vector $V_1$.

As described above, detection system 100 and the detection method according to the first embodiment enable discrimination of the component of one of the two magnetic particles by using the phase difference based on the difference between the relaxation times of the magnetic moments of the two magnetic particles.

When zero magnetic field region 30 is scanned in inspection object 6, steps S11 to S16 are repeatedly performed during scanning of zero magnetic field region 30. Thus, the target component of the target particle is calculated for each position and direction of zero magnetic field region 30 in inspection object 6.

(Spatial Distribution Imaging)

Processor 12 may perform a process of generating an image indicating a spatial distribution where the magnetic particle exists in inspection object 6 (spatial distribution imaging), based on intensity $R_3$ or the target component for each position and direction of zero magnetic field region 30 in inspection object 6. Processor 12 may perform the spatial distribution imaging by using a known successive approximation image reconstruction method.

Fig. 12 is a flowchart showing an example of a flow of the spatial distribution imaging. As shown in Fig. 12, in step S101, processor 12 generates a sinogram (hereinafter referred to as "measured sinogram") from information indicating intensity $R_3$ or the target component for each position and direction of zero magnetic field region 30. The sinogram is a signal map with the horizontal axis representing a scanning position of zero magnetic field region 30 and the vertical axis representing angle $\phi$ (see Fig. 2) indicating the direction of zero magnetic field region 30.

Next, in step S102, processor 12 assumes a distribution of the magnetic particle. In step S103, processor 12 generates an assumed sinogram using the distribution assumed in step S102. In step S104, processor 12 calculates an error between the measured sinogram generated in step S101 and the assumed sinogram generated in step S103. In step S105, processor 12 determines whether the error is equal to or less than a predetermined convergence condition. When the determination of NO is made in step S105, the process returns to step S102.

Processor 12 repeatedly performs the processing in step S102 to step S104 until the error becomes equal to or less than the convergence condition.

When the determination of YES is made in step S105, then in step S106, processor 12 generates data about an image indicating a spatial distribution of the magnetic particle (spatial distribution image data), which corresponds to the assumed sinogram that satisfies the convergence condition, and outputs the generated data. For example, processor 12 causes display unit 16 to display the image indicating the spatial distribution of the magnetic particle.

(Examples of User Interface Screens)

Fig. 13 is a view showing examples of user interface screens provided by the detection system shown in Fig. 1. User interface screens 80, 81 and 82 shown in Fig. 13 are generated by processor 12 and displayed on display unit 16.

User interface screen 80 includes a region 80a and buttons 80b and 80c. A measured sinogram generated from the information indicating intensity $R_3$ for each position and direction of zero magnetic field region 30 is displayed in region 80a.

Button 80b is a button for selecting magnetic particle 51 as the target particle. Button 80c is a button for selecting magnetic particle 52 as the target particle.

When button 80b is pressed, processor 12 causes display unit 16 to display user interface screen 81. User interface screen 81 includes regions 81a and 81b. A measured sinogram generated from the information indicating the target component (i.e., product $Rb_1$ corresponding to magnetic particle 51, which is the target particle) for each position and direction of zero magnetic field region 30 is displayed in region 81a. A spatial distribution image corresponding to an assumed sinogram in which an error between the assumed sinogram and the measured sinogram displayed in region 81a is equal to or less than the convergence condition is displayed in region 81b.

When button 80c is pressed, processor 12 causes display unit 16 to display user interface screen 82. User interface screen 82 includes regions 82a and 82b. A measured sinogram generated from the information indicating the target component (i.e., product $Rb_2$ corresponding to magnetic particle 52, which is the target particle) for each position and direction of zero magnetic field region 30 is displayed in region 82a. A spatial distribution image corresponding to an assumed sinogram in which an error between the assumed sinogram and the measured sinogram displayed in region 82a is equal to or less than the convergence condition is displayed in region 82b.

By checking user interface screen 81, the user can check a distribution of magnetic particle 51 binding to the first protein in inspection object 6. Furthermore, by checking user interface screen 82, the user can check a distribution of magnetic particle 52 binding to the second protein in inspection object 6. As described above, according to the first embodiment, the user can simultaneously check the distributions of the two types of magnetic particles.

Second Embodiment.

A detection system according to a second embodiment is different from detection system 100 according to the first embodiment in that the detection system according to the second embodiment includes a frequency filter, instead of lock-in amplifier 4. In the detection system according to the second embodiment, the frequency filter extracts a signal of a specific frequency from a magnetic signal output from magnetic sensor 3, and outputs the extracted signal. Processor 12 of information processing device 9 acquires the signal of the specific frequency that has passed through the frequency filter, and acquires an intensity and a phase of the signal of the specific frequency.

Third Embodiment.

A detection system according to a third embodiment is different from detection system 100 according to the first embodiment in that the detection system according to the third embodiment does not include lock-in amplifier 4. In the detection system according to the third embodiment, processor 12 of information processing device 9 acquires a signal of a specific frequency by performing fast Fourier transform on a magnetic signal received from magnetic sensor 3 or a signal obtained by amplifying the magnetic signal. Processor 12 acquires an intensity and a phase of the signal of the specific frequency.

Fourth Embodiment.

A detection system according to a fourth embodiment is different from detection system 100 according to the first embodiment in that the detection system according to the fourth embodiment generates and stores phase information indicating phases $\theta_1$ and $\theta_2$, instead of phase information 20 indicating phase difference $\Delta\theta$ and rotation angles $\theta_{rot1}$ and $\theta_{rot2}$.

In the detection system according to the fourth embodiment, in the preliminary preparation phase, processor 12 generates the phase information indicating phases $\theta_1$ and $\theta_2$ and stores the phase information in internal storage unit 15, instead of steps S7 to S9 shown in Fig. 7.

Furthermore, in the measurement phase, processor 12 performs the following process, instead of steps S14 to S16 shown in Fig. 10. Specifically, when magnetic particle 51 is selected as the target particle, processor 12 performs computation in accordance with the following equation (9). When magnetic particle 52 is selected as the target particle, processor 12 performs computation in accordance with the following equation (10). In the equations (9) and (10), n is an integer equal to or more than 0. When n is an even number, processor 12 acquires a real part of a computation result as the target component. When n is an odd number, processor 12 acquires an imaginary part of the computation result as the target component.

[Math. 4]

$$(R_3 \cos\theta_3 + i\,R_3 \sin\theta_3) \times \left\{\cos\left(\theta_2 - \frac{\pi}{2}n\right) - i\sin\left(\theta_2 - \frac{\pi}{2}n\right)\right\} \quad (9)$$

$$(R_3 \cos\theta_3 + i\,R_3 \sin\theta_3) \times \left\{\cos\left(\theta_1 - \frac{\pi}{2}n\right) - i\sin\left(\theta_1 - \frac{\pi}{2}n\right)\right\} \quad (10)$$

Fifth Embodiment.

Fig. 14 is a view showing two types of magnetic particles detected by a detection system according to a fifth embodiment. As shown in Fig. 14, the detection system according to the fifth embodiment detects magnetic particles 53 and 54 instead of magnetic particles 51 and 52, as compared with detection system 100 according to the first embodiment. Magnetic particle 53 is an example of "first magnetic particle" in the present disclosure. Magnetic particle 54 is an example of "second magnetic particle" in the present disclosure.

Magnetic particles 53 and 54 have different states of binding to a target substance 62 in inspection object 6. That is, magnetic particle 53 does not bind to target substance 62. Magnetic particle 54 binds to target substance 62. When magnetic particles 53 and 54 have particle sizes where the Brownian motion is dominant, magnetic particles 53 and 54 have different Brownian relaxation times. Therefore, when an excitation magnetic field is applied, a phase of a signal of a specific frequency generated in response to a change in magnetic moment of magnetic particle 53 is different from a phase of a signal of the specific frequency generated in response to a change in magnetic moment of magnetic particle 54. Therefore, the detection system according to the fifth embodiment can use the same method as the detection method according to the first embodiment to discriminate a component of a target particle, of magnetic particles 53 and 54, from a signal of the specific frequency generated in response to a change in magnetic moment of a zero magnetic field region including magnetic particles 53 and 54.

It should be understood that the embodiments disclosed herein are illustrative and non-restrictive in every respect. The scope of the present disclosure is defined by the terms of the claims, rather than the description of the embodiments above, and is intended to include any modifications within the scope and meaning equivalent to the terms of the claims.

Hereinafter, various aspects of the present disclosure will be summarized as additional aspects.

(Additional Aspect 1)

A detection method for detecting a magnetic particle by using an AC excitation magnetic field, the detection method comprising:

acquiring a first phase of a first signal of a specific frequency generated in response to a change in magnetic moment of a first magnetic particle when the excitation magnetic field is applied;
acquiring a second phase of a second signal of the specific frequency generated in response to a change in magnetic moment of a second magnetic particle when the excitation magnetic field is applied, a relaxation time of the magnetic moment of the second magnetic particle being different from a relaxation time of the magnetic moment of the first magnetic particle at the specific frequency;
acquiring a third signal of the specific frequency generated in response to a change in magnetic moment of a target region including the first magnetic particle and the second magnetic particle when the excitation magnetic field is applied; and
discriminating a target component of a target particle, of the first magnetic particle and the second magnetic particle, from the third signal by using the first phase and the second phase.

(Additional Aspect 2)

The detection method according to Additional Aspect 1, wherein

when the target particle is the first magnetic particle, the target component is calculated by using an intensity of a component having a phase orthogonal to the second phase in the third signal, and
when the target particle is the second magnetic particle, the target component is calculated by using an intensity of a component having a phase orthogonal to the first phase in the third signal.

(Additional Aspect 3)

The detection method according to Additional Aspect 2, wherein
the target component represents a product of the intensity and 1/sin ($\Delta\theta$), and the $\Delta\theta$ is a difference between the first phase and the second phase.

(Additional Aspect 4)

The detection method according to any one of Additional Aspects 1 to 3, wherein
the first magnetic particle and the second magnetic particle have different particle sizes.

(Additional Aspect 5)

The detection method according to any one of Additional Aspects 1 to 3, wherein
the first magnetic particle and the second magnetic particle have different states of binding to a target substance.

(Additional Aspect 6)

The detection method according to any one of Additional Aspects 1 to 5, further comprising
selecting the target particle from the first magnetic particle and the second magnetic particle in accordance with an input.

(Additional Aspect 7)

The detection method according to any one of Additional Aspects 1 to 6, further comprising:

scanning the target region in an inspection object including the first magnetic particle and the second magnetic particle; and
generating an image indicating a spatial distribution of the target particle in the inspection object, based on the target component.

(Additional Aspect 8)

A detection system comprising:

an excitation magnetic field applicator that applies an AC excitation magnetic field to a target region;
a magnetic sensor that detects a magnetic signal indicating a change in magnetic moment of the target region; and
a processor, wherein
the processor

acquires a first phase of a first signal of a specific frequency extracted from the magnetic signal when a first magnetic particle is placed in the target region,
acquires a second phase of a second signal of the specific frequency extracted from the magnetic signal when a second magnetic particle is placed in the target region, a relaxation time of a magnetic moment of the second magnetic particle being different from a relaxation time of a magnetic moment of the first magnetic particle at the specific frequency,
acquires a third signal of the specific frequency extracted from the magnetic signal when an inspection object including the first magnetic particle and the second magnetic particle is placed in the target region, and
discriminates a target component of a target particle, of the first magnetic particle and the second magnetic particle, from the third signal by using the first phase and the second phase.

(Additional Aspect 9)

The detection system according to Additional Aspect 8, wherein

when the target particle is the first magnetic particle, the processor calculates the target component by using an intensity of a component having a phase orthogonal to the second phase in the third signal, and
when the target particle is the second magnetic particle, the processor calculates the target component by using an intensity of a component having a phase orthogonal to the first phase in the third signal.

(Additional Aspect 10)

The detection system according to Additional Aspect 9, wherein
the target component represents a product of the intensity and $1/\sin(\Delta\theta)$, and the $\Delta\theta$ is a difference between the first phase and the second phase.

(Additional Aspect 11)

The detection system according to any one of Additional Aspects 8 to 10, wherein
the processor selects the target particle from the first magnetic particle and the second magnetic particle in accordance with an input.

(Additional Aspect 12)

The detection system according to any one of Additional Aspects 8 to 11, wherein
the processor generates an image indicating a spatial distribution of the target particle in the inspection object, based on the target component when the processor scans the target region in the inspection object.

(Additional Aspect 13)

A program that causes a computer to perform a detection method for detecting a magnetic particle by using an AC excitation magnetic field,
the detection method comprising:

acquiring a first phase of a first signal of a specific frequency generated in response to a change in magnetic moment of a first magnetic particle when the excitation magnetic field is applied;
acquiring a second phase of a second signal of the specific frequency generated in response to a change in magnetic moment of a second magnetic particle when the excitation magnetic field is applied, a relaxation time of the magnetic moment of the second magnetic particle being different from a relaxation time of the magnetic moment of the first magnetic particle at the specific frequency;
acquiring a third signal of the specific frequency generated in response to a change in magnetic moment of a target region including the first magnetic particle and the second magnetic particle when the excitation magnetic field is applied; and
discriminating a target component of a target particle, of the first magnetic particle and the second magnetic particle, from the third signal by using the first phase and the second phase.

(Additional Aspect 14)

A computer-readable recording medium having the program according to Additional Aspect 13 recorded thereon.

REFERENCE SIGNS LIST

1 excitation magnetic field applicator; 2 zero magnetic field generator; 2a, 2b electromagnet; 3 magnetic sensor; 4 lock-in amplifier; 5 measurement area; 6 inspection object; 7 first power supply; 8a second power supply; 8b third power supply; 9 information processing device; 10 detection program; 11 optical recording medium; 12 processor; 13 RAM; 14 reading unit; 15 internal storage unit; 16 display unit; 17 operation unit; 18 communication interface; 19 server device; 20 phase information; 30 zero magnetic field region; 41, 42 mixer; 43, 44 low-pass filter; 45 calculator; 51 to 54 magnetic particle; 51a, 52a probe; 60 tissue; 62 target substance; 70, 72 axis; 80, 81, 82 user interface screen; 80a, 81a, 81b, 82a, 82b region; 80b, 80c button; 100 detection system.

## Claims

**1.** A detection method for detecting a magnetic particle by using an AC excitation magnetic field, the detection method comprising:

acquiring a first phase of a first signal of a specific frequency generated in response to a change in magnetic moment of a first magnetic particle when the excitation magnetic field is applied;
acquiring a second phase of a second signal of the specific frequency generated in response to a change in magnetic moment of a second magnetic particle when the excitation magnetic field is applied, a relaxation time of the magnetic moment of the second magnetic particle being different from a relaxation time of the magnetic moment of the first magnetic particle at the specific frequency;
acquiring a third signal of the specific frequency generated in response to a change in magnetic moment of a target region including the first magnetic particle and the second magnetic particle when the excitation magnetic field is applied; and
discriminating a target component of a target particle, of the first magnetic particle and the second magnetic particle, from the third signal by using the first phase and the second phase.

**2.** The detection method according to claim 1, wherein

when the target particle is the first magnetic particle, the target component is calculated by using an intensity of a component having a phase orthogonal to the second phase in the third signal, and
when the target particle is the second magnetic particle, the target component is calculated by using an intensity of a component having a phase orthogonal to the first phase in the third signal.

**3.** The detection method according to claim 2, wherein
the target component represents a product of the intensity and 1/sin ($\Delta\theta$), and the $\Delta\theta$ is a difference between the first phase and the second phase.

**4.** The detection method according to any one of claims 1 to 3, wherein
the first magnetic particle and the second magnetic particle have different particle sizes.

**5.** The detection method according to any one of claims 1 to 3, wherein
the first magnetic particle and the second magnetic particle have different states of binding to a target substance.

**6.** The detection method according to any one of claims 1 to 5, further comprising

selecting the target particle from the first magnetic particle and the second magnetic particle in accordance with an input.

**7.** The detection method according to any one of claims 1 to 6, further comprising:

scanning the target region in an inspection object including the first magnetic particle and the second magnetic particle; and
generating an image indicating a spatial distribution of the target particle in the inspection object, based on the target component.

**8.** A detection system comprising:

an excitation magnetic field applicator that applies an AC excitation magnetic field to a target region;
a magnetic sensor that detects a magnetic signal indicating a change in magnetic moment of the target region; and
a processor, wherein
the processor

acquires a first phase of a first signal of a specific frequency extracted from the magnetic signal when a first magnetic particle is placed in the target region,
acquires a second phase of a second signal of the specific frequency extracted from the magnetic signal when a second magnetic particle is placed in the target region, a relaxation time of a magnetic moment of the second magnetic particle being different from a relaxation time of a magnetic moment of the first magnetic particle at the specific frequency,
acquires a third signal of the specific frequency extracted from the magnetic signal when an inspection object including the first magnetic particle and the second magnetic particle is placed in the target region, and
discriminates a target component of a target particle, of the first magnetic particle and the second magnetic particle, from the third signal by using the first phase and the second phase.

**9.** The detection system according to claim 8, wherein

when the target particle is the first magnetic particle, the processor calculates the target component by using an intensity of a component having a phase orthogonal to the second phase in the third signal, and
when the target particle is the second magnetic particle, the processor calculates the target component by using an intensity of a component having a phase orthogonal to the first phase in the third signal.

**10.** The detection system according to claim 9, wherein
the target component represents a product of the intensity and 1/sin ($\Delta\theta$), and the $\Delta\theta$ is a difference between the first phase and the second phase.

**11.** The detection system according to any one of claims 8 to 10, wherein
the processor selects the target particle from the first magnetic particle and the second magnetic particle in accordance with an input.

**12.** The detection system according to any one of claims 8 to 11, wherein
the processor generates an image indicating a spatial distribution of the target particle in the inspection object, based on the target component when the processor scans the target region in the inspection object.

**13.** A program that causes a computer to perform a detection method for detecting a magnetic particle by using an AC excitation magnetic field,
the detection method comprising:

acquiring a first phase of a first signal of a specific frequency generated in response to a change in magnetic moment of a first magnetic particle when the excitation magnetic field is applied;
acquiring a second phase of a second signal of the specific frequency generated in response to a change in magnetic moment of a second magnetic particle when the excitation magnetic field is applied, a relaxation time of the magnetic moment of the second magnetic particle being different from a relaxation time of the magnetic moment of the first magnetic particle at the specific frequency;

acquiring a third signal of the specific frequency generated in response to a change in magnetic moment of a target region including the first magnetic particle and the second magnetic particle when the excitation magnetic field is applied; and

discriminating a target component of a target particle, of the first magnetic particle and the second magnetic particle, from the third signal by using the first phase and the second phase.

**14.** A computer-readable recording medium having the program according to claim 13 recorded thereon.

FIG.1

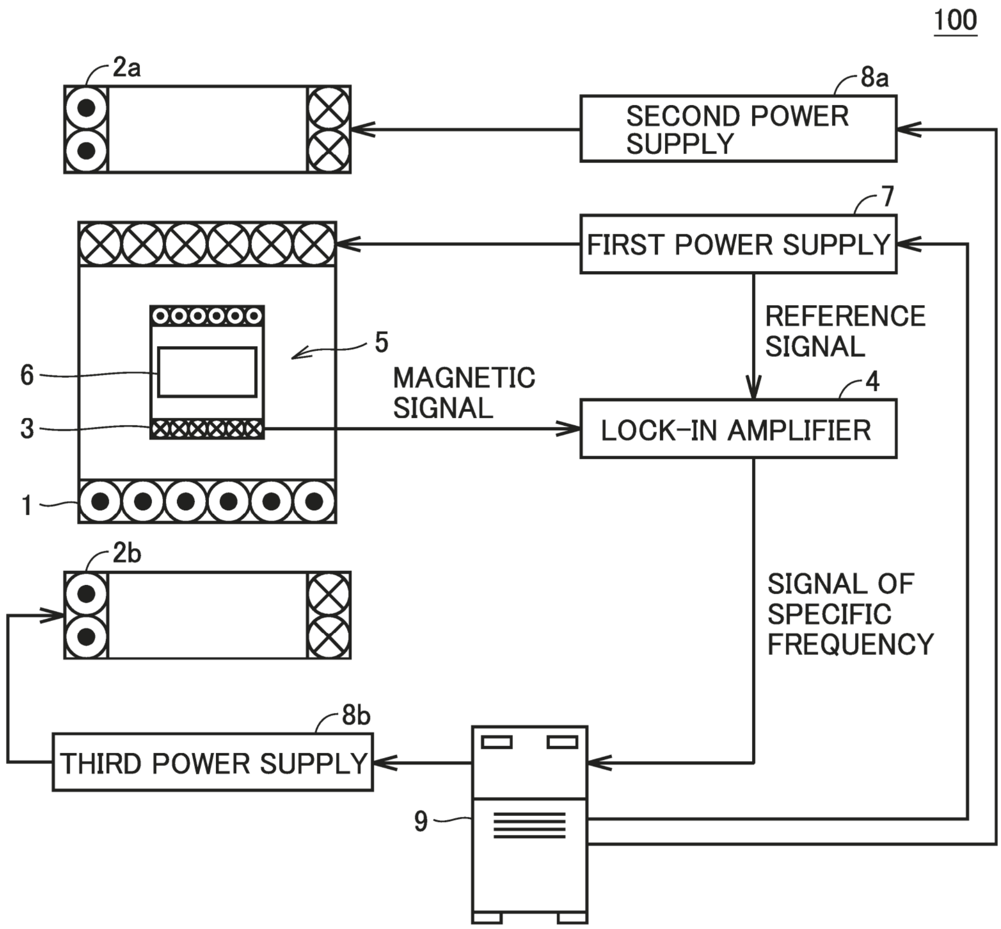
100
2a
8a
SECOND POWER SUPPLY
7
FIRST POWER SUPPLY
REFERENCE SIGNAL
5
6
MAGNETIC SIGNAL
4
3
LOCK-IN AMPLIFIER
1
2b
SIGNAL OF SPECIFIC FREQUENCY
8b
THIRD POWER SUPPLY
9

FIG.2

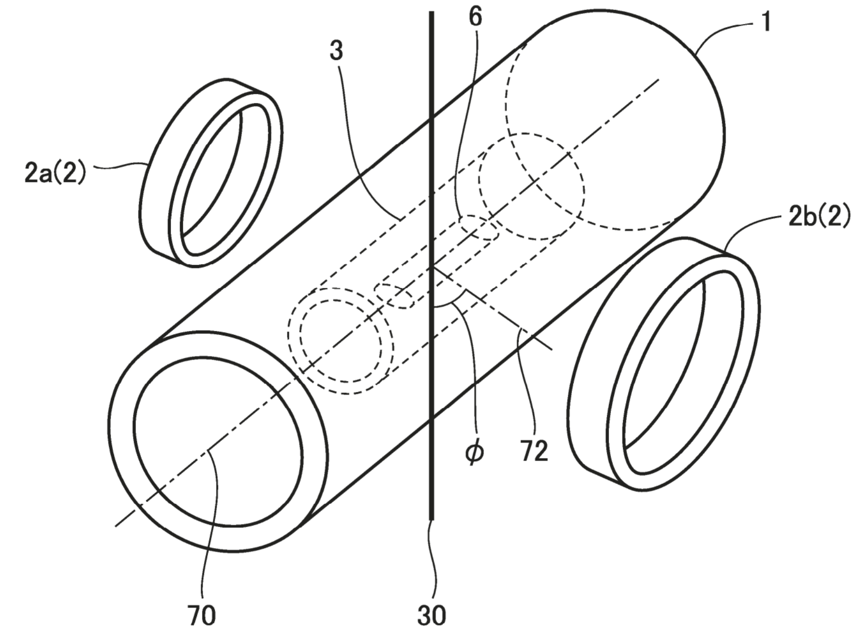

FIG.3

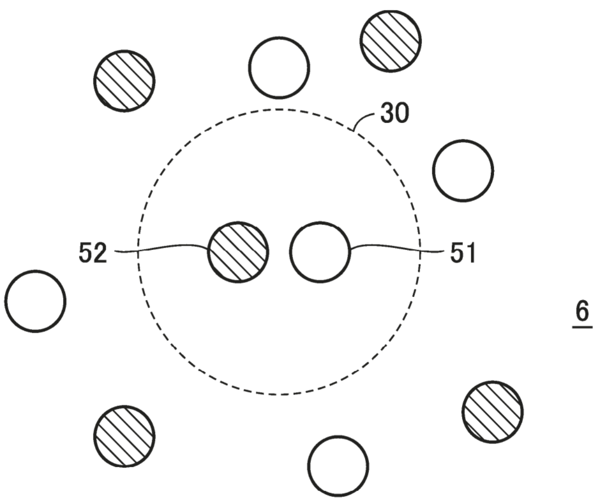

FIG.4

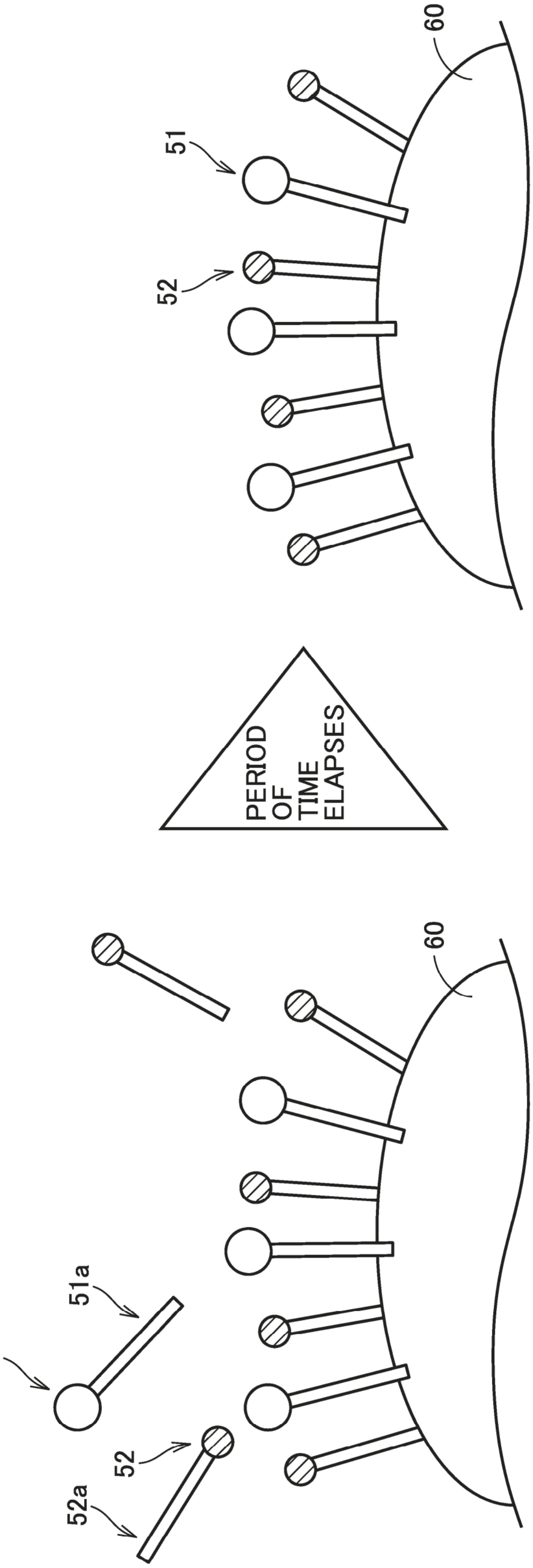

FIG.5

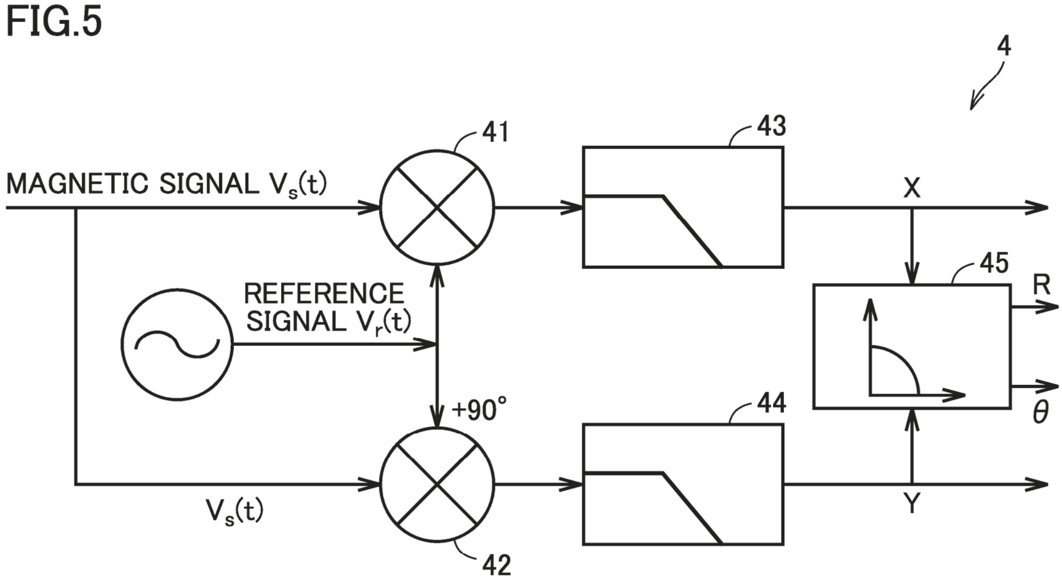
4
41
43
MAGNETIC SIGNAL $V_s(t)$
X
45
R
REFERENCE
SIGNAL $V_r(t)$
+90°
44
θ
$V_s(t)$
Y
42

FIG.6

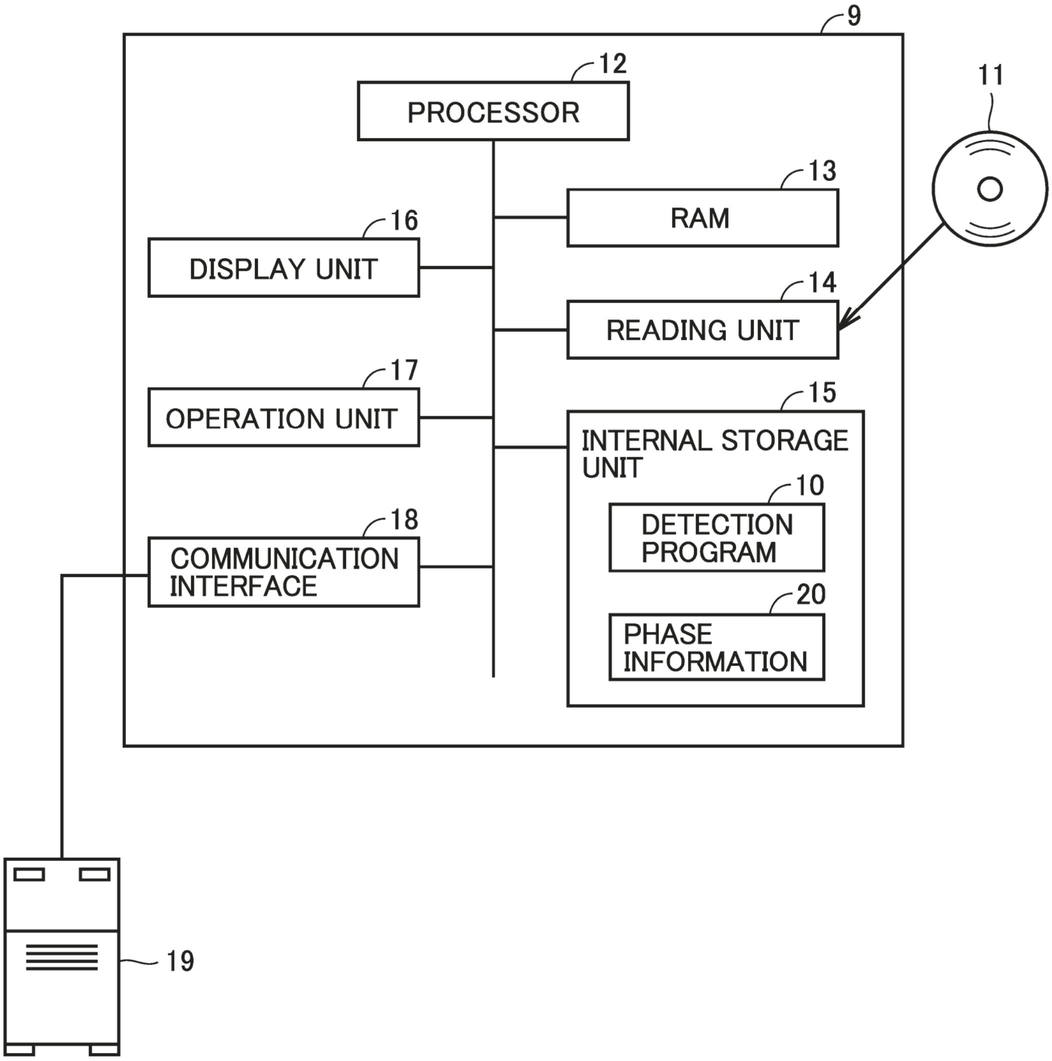
9
12
PROCESSOR
11
13
RAM
16
DISPLAY UNIT
14
READING UNIT
17
OPERATION UNIT
15
INTERNAL STORAGE UNIT
10
DETECTION PROGRAM
18
COMMUNICATION INTERFACE
20
PHASE INFORMATION
19

FIG.7

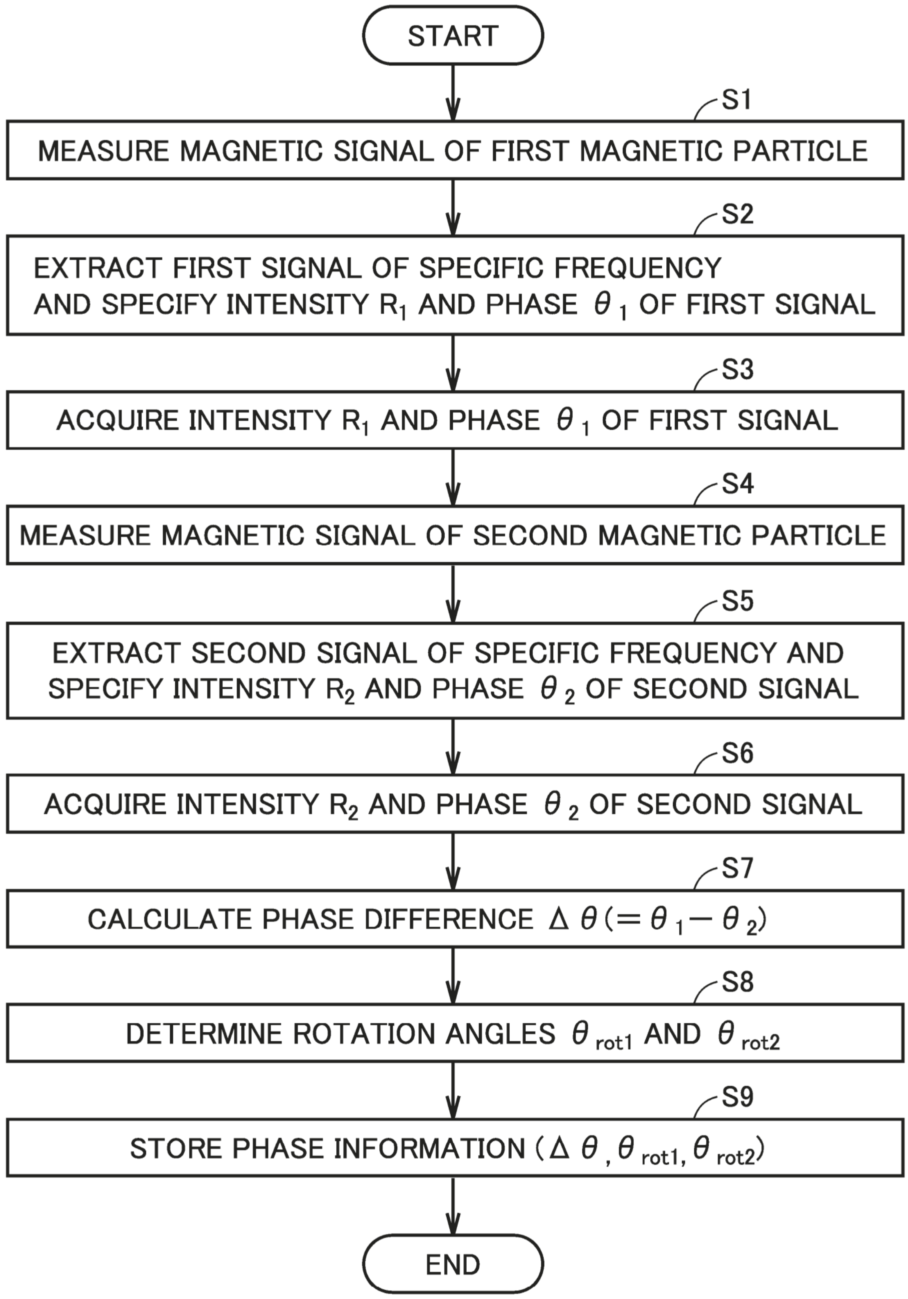
START
S1
MEASURE MAGNETIC SIGNAL OF FIRST MAGNETIC PARTICLE
S2
EXTRACT FIRST SIGNAL OF SPECIFIC FREQUENCY AND SPECIFY INTENSITY $R_1$ AND PHASE $\theta_1$ OF FIRST SIGNAL
S3
ACQUIRE INTENSITY $R_1$ AND PHASE $\theta_1$ OF FIRST SIGNAL
S4
MEASURE MAGNETIC SIGNAL OF SECOND MAGNETIC PARTICLE
S5
EXTRACT SECOND SIGNAL OF SPECIFIC FREQUENCY AND SPECIFY INTENSITY $R_2$ AND PHASE $\theta_2$ OF SECOND SIGNAL
S6
ACQUIRE INTENSITY $R_2$ AND PHASE $\theta_2$ OF SECOND SIGNAL
S7
CALCULATE PHASE DIFFERENCE $\Delta\theta (= \theta_1 - \theta_2)$
S8
DETERMINE ROTATION ANGLES $\theta_{rot1}$ AND $\theta_{rot2}$
S9
STORE PHASE INFORMATION $(\Delta\theta, \theta_{rot1}, \theta_{rot2})$
END

FIG.8

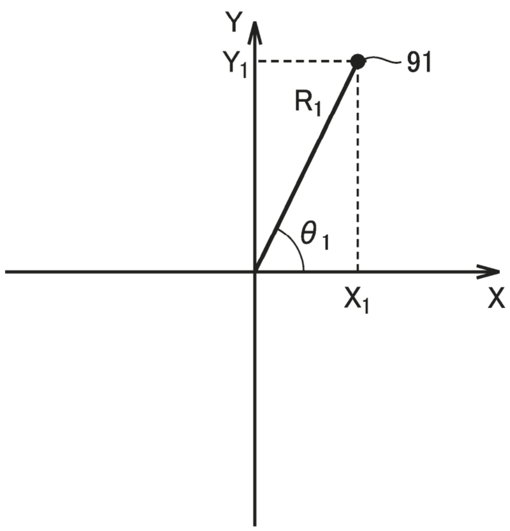

FIG.9

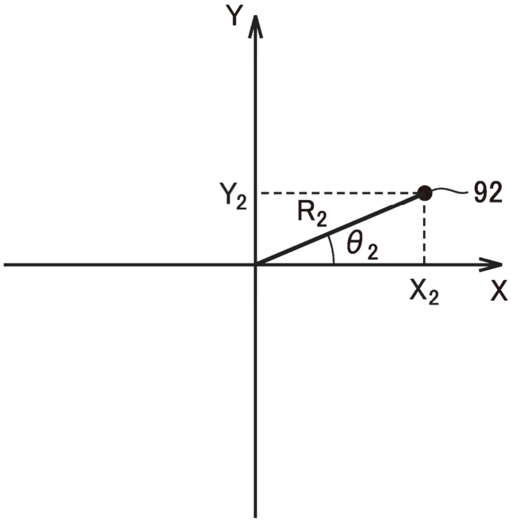
Y
Y2
R2
θ2
92
X2
X

FIG.10

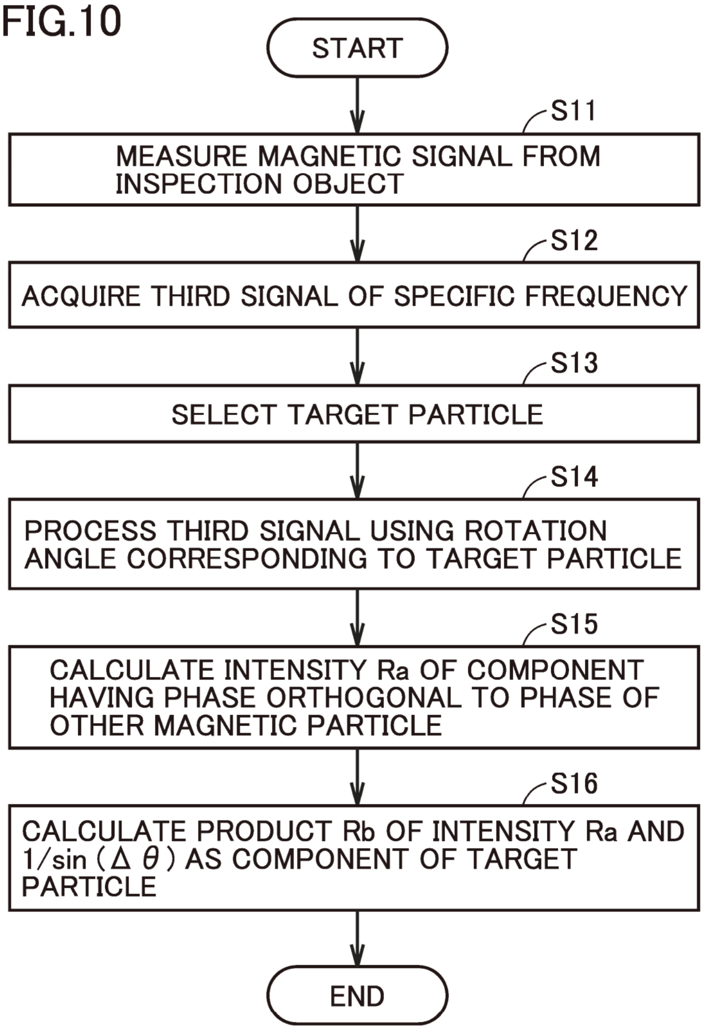
START
S11
MEASURE MAGNETIC SIGNAL FROM INSPECTION OBJECT
S12
ACQUIRE THIRD SIGNAL OF SPECIFIC FREQUENCY
S13
SELECT TARGET PARTICLE
S14
PROCESS THIRD SIGNAL USING ROTATION ANGLE CORRESPONDING TO TARGET PARTICLE
S15
CALCULATE INTENSITY Ra OF COMPONENT HAVING PHASE ORTHOGONAL TO PHASE OF OTHER MAGNETIC PARTICLE
S16
CALCULATE PRODUCT Rb OF INTENSITY Ra AND 1/sin (Δ θ) AS COMPONENT OF TARGET PARTICLE
END

FIG.11

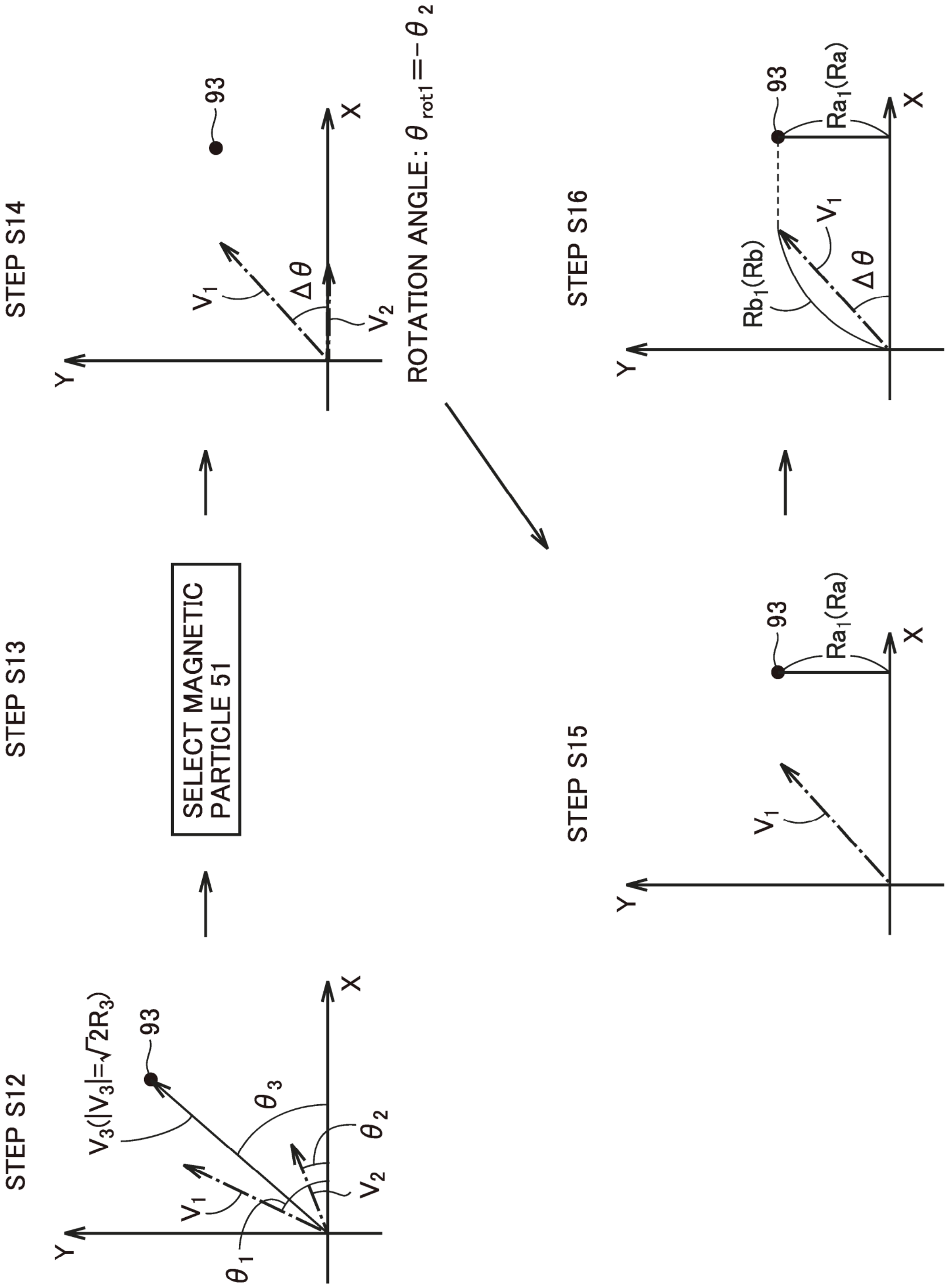
STEP S12
STEP S13
STEP S14
STEP S15
STEP S16
SELECT MAGNETIC PARTICLE 51
ROTATION ANGLE: $\theta_{rot1} = -\theta_2$
93
$V_3(|V_3| = \sqrt{2}R_3)$
$V_1$
$V_2$
$\theta_1$
$\theta_2$
$\theta_3$
$\Delta\theta$
$Ra_1(Ra)$
$Rb_1(Rb)$
X
Y

FIG.12

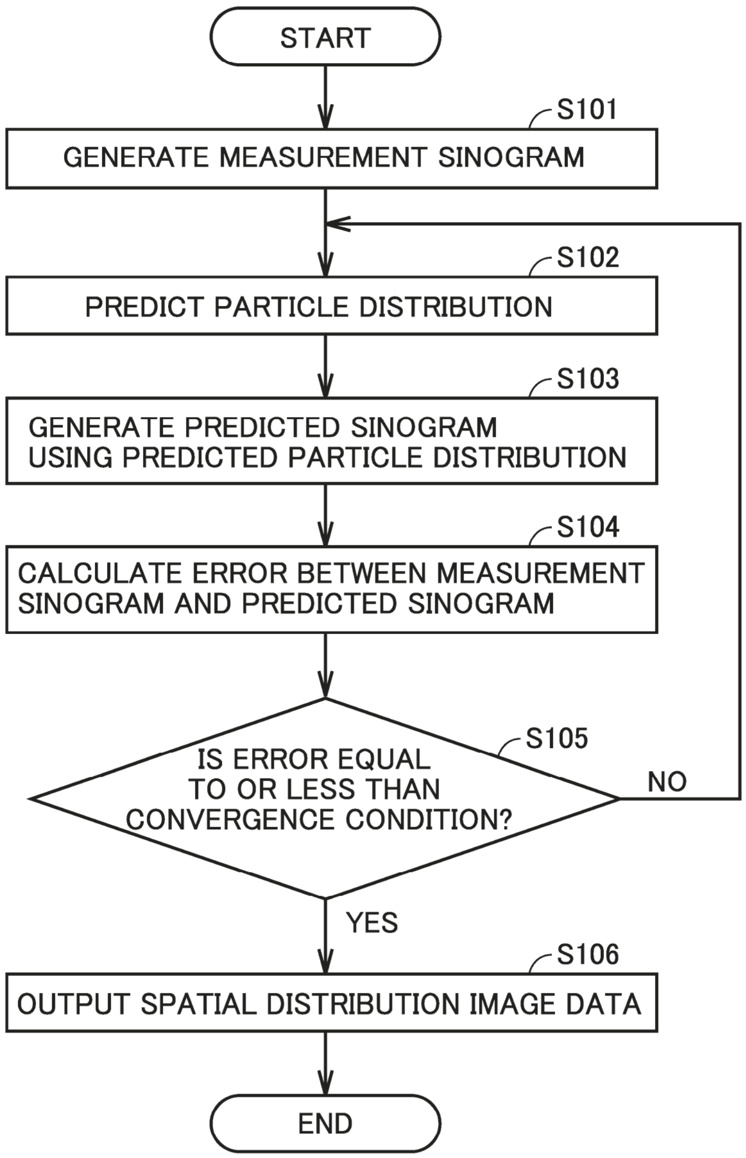
START
S101
GENERATE MEASUREMENT SINOGRAM
S102
PREDICT PARTICLE DISTRIBUTION
S103
GENERATE PREDICTED SINOGRAM USING PREDICTED PARTICLE DISTRIBUTION
S104
CALCULATE ERROR BETWEEN MEASUREMENT SINOGRAM AND PREDICTED SINOGRAM
S105
IS ERROR EQUAL TO OR LESS THAN CONVERGENCE CONDITION?
NO
YES
S106
OUTPUT SPATIAL DISTRIBUTION IMAGE DATA
END

FIG.13

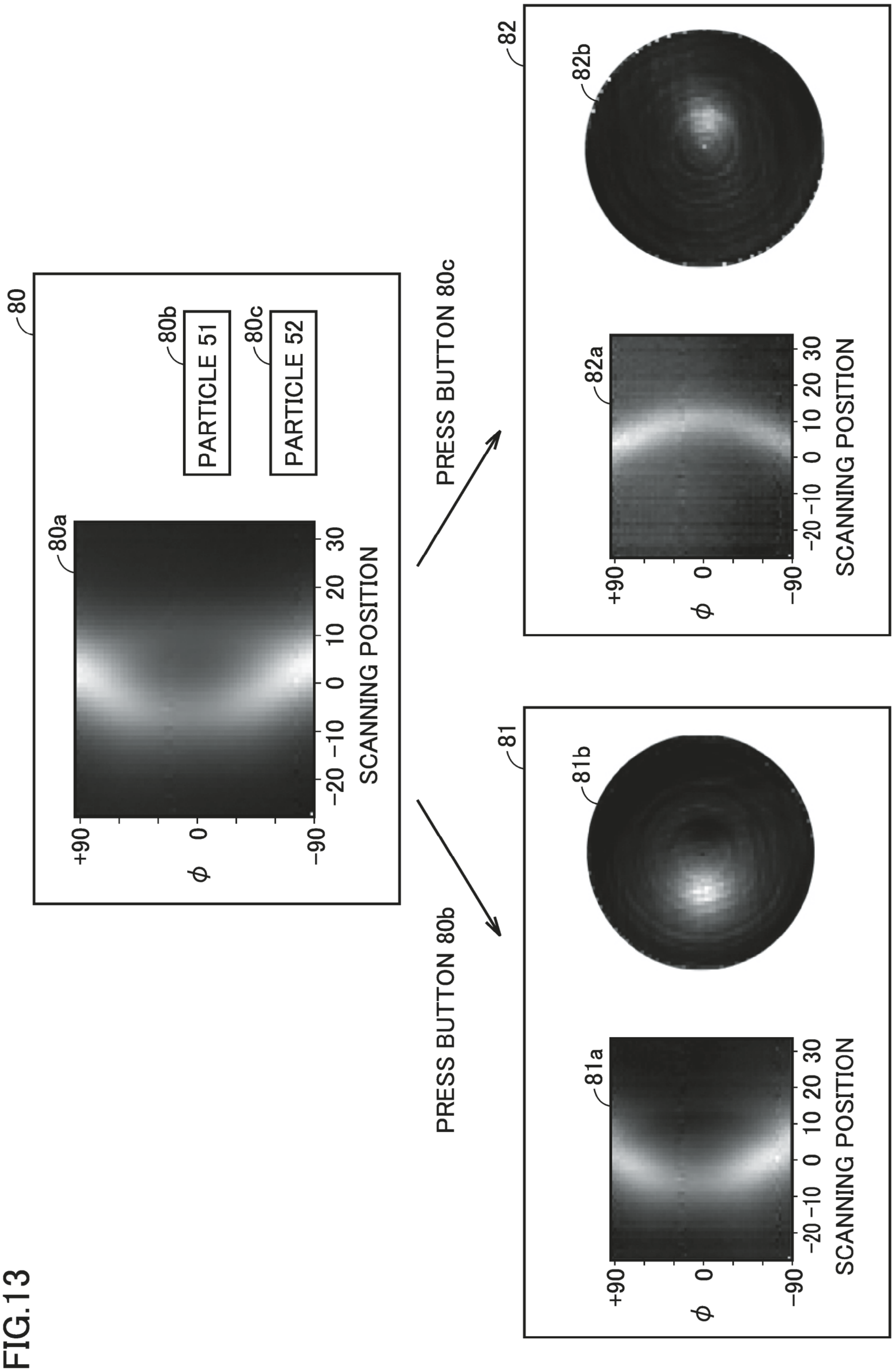
80
80a
φ
+90
0
−90
−20 −10 0 10 20 30
SCANNING POSITION
80b
PARTICLE 51
80c
PARTICLE 52
PRESS BUTTON 80b
PRESS BUTTON 80c
81
81a
81b
82
82a
82b

FIG.14

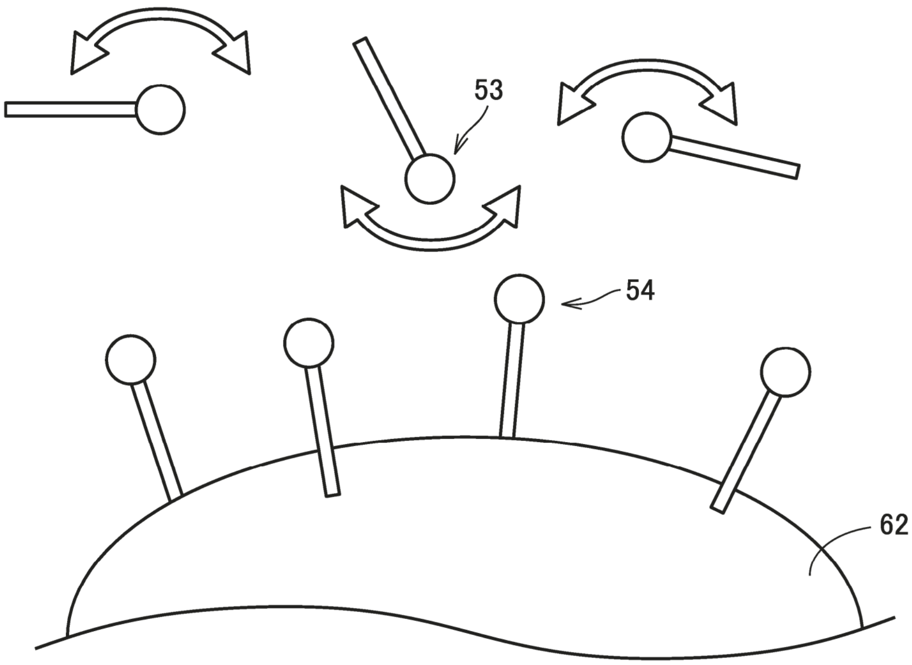

**INTERNATIONAL SEARCH REPORT**

International application No.
**PCT/JP2024/016706**

**A. CLASSIFICATION OF SUBJECT MATTER**

***G01N 27/72***(2006.01)i
FI: G01N27/72

According to International Patent Classification (IPC) or to both national classification and IPC

**B. FIELDS SEARCHED**

Minimum documentation searched (classification system followed by classification symbols)

A61B5/05-A61B5/0538; A61B5/24-A61B5/398; G01N27/72-G01N27/9093; G01R33/00-G01R33/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2022/224417 A1 (MITSUBISHI ELECTRIC CORPORATION) 27 October 2022 (2022-10-27)<br>paragraphs [0016]-[0080], fig. 1-14 | 1-14 |
| A | JP 2021-67687 A (IMRA AMERICA INC.) 30 April 2021 (2021-04-30) | 1-14 |
| A | WO 2012/011477 A1 (HITACHI, LTD.) 26 January 2012 (2012-01-26) | 1-14 |
| A | JP 2013-228280 A (HITACHI, LTD.) 07 November 2013 (2013-11-07) | 1-14 |
| A | JP 2007-538252 A (KONINKLIJKE PHILIPS ELECTRONICS N.V.) 27 December 2007 (2007-12-27) | 1-14 |
| A | WO 2012/046157 A1 (KONINKLIJKE PHILIPS ELECTRONICS N.V.) 12 April 2012 (2012-04-12) | 1-14 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 May 2024** | **04 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016706**

| Patent document cited in search report | Publication date (day/month/year) | Patent family member(s) | Publication date (day/month/year) |
|---|---|---|---|
| WO 2022/224417 A1 | 27 October 2022 | CN 117279564 A | |
| JP 2021-67687 A | 30 April 2021 | US 2022/0178919 A1 | |
| WO 2012/011477 A1 | 26 January 2012 | US 2013/0121879 A1 | |
| JP 2013-228280 A | 07 November 2013 | US 2013/0288384 A1 | |
| JP 2007-538252 A | 27 December 2007 | US 2007/0172890 A1<br>WO 2005/111596 A1<br>CN 1957251 A | |
| WO 2012/046157 A1 | 12 April 2012 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003199767 A

### Non-patent literature cited in the description

- **GLEICH, B** ; **WEIZENECKER, J**. Tomographic imaging using the nonlinear response of magnetic particles. *nature*, 2005, vol. 435, 1214-1217
- **KENYA MURASE et al.** Development of a system for magnetic particle imaging using neodymium magnets and gradiometer. *Japanese Journal of Applied Physics*, 2014, vol. 53, 067001